Europäisches Patentamt

European Patent Office

Office européen des brevets

(11) Publication number: **0 332 298**
**A2**

# EUROPEAN PATENT APPLICATION

(21) Application number: 89301348.2

(51) Int. Cl.4: **C07H 15/10**

(22) Date of filing: **13.02.89**

(30) Priority: **12.02.88 US 155401**
**15.08.88 US 232219**

(43) Date of publication of application:
**13.09.89 Bulletin 89/37**

(84) Designated Contracting States:
**AT BE CH DE ES FR GB GR IT LI LU NL SE**

(71) Applicant: **The Biomembrane Institute**
**201 Elliott Avenue West Suite 305**
**Seattle Washington 98119(US)**

(72) Inventor: **Hakomori, Sen-itiroh**
**The Biomembrane Institute 201 Elliott**
**Avenue West**
**Seattle, WA 98119(US)**
Inventor: **Nores, Gustavo**
**The Biomembrane Institute 201 Elliott**
**Avenue West**
**Seattle, WA 98119(US)**
Inventor: **Hanai, Nobuo**

The Biomembrane Institute 201 Elliott
Avenue West
Seattle, WA 98119(US)
Inventor: **Dohi, Taeko**
The Biomembrane Institute 201 Elliott
Avenue West
Seattle, WA 98119(US)
Inventor: **Levery, Steven B.**
The Biomembrane Institute 201 Elliott
Avenue West
Seattle, WA 98119(US)
Inventor: **Salyan, Mary Ellen K.**
The Biomembrane Institute 201 Elliott
Avenue West
Seattle, WA 98119(US)

(74) Representative: **Woodcraft, David Charles et**
**al**
**BROOKES & MARTIN High Holborn House**
**52/54 High Holborn**
**London, WC1V 6SE(GB)**

(54) **Methods of preparing de-N-acylated and N-acylated glycospingo lipids and lyso forms of glycospingo lipids.**

(57) A method for preparing de-N-acylated forms of an N-acyl sugar-containing glycospingolipid comprising hydrolyzing the glycospingolipids under mild alkaline conditions such that the N-acyl group of the sugar moiety is preferentially hydrolyzed. A method for preparing N-acylated forms of an amino sugar-containing lyso form glycospingolipid comprising protecting the amino group of the spingosine by incorporating the glycospingolipid into a phospholipid or hydrophobic matrix and then conducting selective N-acylation on the amino group of the sugar moiety. Methods for preparing labeled glycospingolipids also disclosed.

FIG.2

## METHODS FOR PREPARING DE-N-ACYLATED AND N-ACYLATED GLYCOSPINGOLIPIDS AND LYSO FORMS OF GLYCOSPINGOLIPIDS

The present invention relates to a method of preparing de-N-acyl glycospingolipids from N-acylated glycospingolipids and to methods of preparing lyso forms of glycospingolipids from N-acylated glycospingolipids and amino sugar-containing lyso form glycospingolipids. The invention also relates to use of the above compounds to prepare labeled, e.g. radiolabeled, glycospingolipids and lyso form glycospingolipids.

Glycospingolipids are an important class of glycolipids and can be regarded as glycosides of N-acylspingosine. These glycospingolipids are generally divided into three classes: (1) neutral glycolipids, (2) sulfatides (sulfate-containing glycolipids), and (3) gangliosides (sialic acid-containing glycolipids).

Glycospingolipids are also classified according to the number of sugar residues and according to the oligosaccharide core structures.

Glycospingolipids and, in particualr, gangliosides are of great interest due to their activity as modifiers of cell physiology (E. Bremer, et a., J. Biol. Chem., 261 (1986) 2434-2440; S. Hakomori, et al., in G. Tettamanti, R. Ledeen, Y. Nagai, K. Sandhoff, and G. Toffano (Eds.), Neuronal plasticity and gangliosides, Liviana Press, Pavoda, 1986, pp. 201-214; and N. Hanai et al., Biochem. Biophys. Res. Commun., 147 (1987) 127-134). Therefore, the synthesis of various glycospingolipid derivatives and analogues is an important area of research.

The lyso form of $GM_3$ has been prepared (T. Taketomi and N. Kawamura, J. Biochem. (Tokyo), 68 (1970) 475-485) by treatment of $GM_3$ with a refluxing solution of 1 M KOH in aqueous 90% butanol for 2.5 h. The product was claimed to have a strong hemolytic activity (twice that of lysolecithin) and was called lysosphingolipid or lysohematoside. The compound, however, had free amino groups at both neuraminic acid and sphingosine. (The term "neuraminic acid" is used according to the original definition (G. Blix, et al., Nature. 179 (1957) 1088), i.e., de-N-acyl sialic acid is defined as "neuraminic acid", while N-acetyl, N-glycolyl and O-acyl derivatives of neuraminic acid are collectively called "sialic acid.")

More recently, various gangliosides ($GM_3$, $GM_2$, $GM_1$, and $GD_{1a}$) have been derivatized (S. Neuenhofer, G. et al, Biochemistry, 24 (1985) 525-532) into their lyso forms, in which the amino groups of neuraminic acid and hexosamines were N-acetylated and only the amino group of the sphingosine was unsubstituted. For this derivatization, the amino group of the sphingosine was first blocked by a hydrophobic protective group (9-fluorenylmethoxycarbonyl), followed by acetylation of the amino groups of neuraminic acid and hexosamines, and subsequent removal of the protective group by liquid ammonia. The procedure involves several steps, and the yield is poor (about 30%).

Some embodiments of the present invention provide new methods of preparing de-N-acylated and N-acylated glycospingolipids and lyso form glycospingolipids which involve few steps and give a high yield.

Some embodiments of the present invention provide novel methods of producing labeled glycospingolipids.

According to the invention there is provided a method for preparing de-N-acylated forms of an N-acyl amino sugar and sialic acid containing glycospingolipid comprising hydrolyzing the glycospingolipids under mild alkaline conditions such that the N-acyl group of the amino sugar and sialic acid moiety is preferentially hydrolyzed.

The present invention also provides a method for preparing labeled N-acylated forms of amino sugar-containing glycospingolipids comprising N-acylating the amino group on the sugar moiety with a labeled N-acylation reagent.

In another aspect, the present invention provides a method for preparing N-acylated forms of an amino sugar-containing lyso form glycospingolipid comprising protecting the amino group of the spingosine by incorporating the glycospingolipid into a phospholipid or hydrophobic matrix and then conducting selective N-acylation on the amino group of the sugar moiety.

In an even further aspect, the present invention provides a method of preparing labeled N-acylated forms of an amino-sugar containing lyso form glycospingolipid comprising protecting the amino group of the spingosine by incorporating the glycospingolipid into a phospholipid or hydrophobic matrix and then conducting selective N-acylation on the amino group of the sugar moiety with a labeled N-acylation reagent.

The present invention further provides a method for preparing labeled glycospingolipids from an acylated sugar-containing lyso form glycospingolipid comprising acylating the amino group on the spingosine moiety with a fatty acid containing labeled groups.

The above-described methods are especially applicable to gangliosides.

The invention will be illustrated by reference to the accompanying drawings of which;

Figure 1 shows the structures of GM₃, lyso-GM₃, and de-N-acetyl-GM₃. The glycolipids are abbreviated according to the system of Svennerholm (Svennerholm, L., J Neurochem., 10 (1963) 613-623) for ganglio-series gangliosides.

Figure 2 is a schematic diagram of the various methods according to the present invention as described in the Examples with respect to the ganglioside GM₃.

Figure 3 is a high-performance thin layer chromatography pattern of the derivatives of GM₃ using chloroform-methanol-aqueous 0.2% CaCl₂ (5:4:1) and detection with resorcinol. D₃ = lyso-GM₃, D₁ = de-N-acetyl-GM₃, and D₂ = de-N-acetyl-lyso-GM₃.

Figure 4 is a high-performance thin-layer chromatography pattern of the products of carbodiimide-catalyzed N-acetylation of de-N-acetyl-lyso-GM₃ (D₂) in micellar dispersion.

Figure 5 is a proton labeling scheme for MNR analysis of GM₃.

Figure 6 show resolution enhanced ¹H-NMR spectra: (A) GM₃, (B) lyso-GM₃ (D₃), (C) de-N-acetyl-GM₃ (D₁) and (D) de-N-acetyl-lyso-GM₃ (D₂). The resonance assignments given in (A) follow those of Koerner et al (T.A.W. Koerner, Jr., et al (1983) Biochemistry 22, 2676-2690). The peaks a-c are due to HOD Me₂SO, and acetone, respectively.

Figure 7 is a negative ion f.a.b.-mass spectra of dog erythrocyte GM₃ in A: triethanolamine (TEA) matrix, B: glycerol matrix. Ions are designated as in Table I: a: triethanolamine matrix cluster ions of formula (n[TEA]-1)⁻, b: glycerol matrix cluster ions of formula (nG-1)⁻, wherein G represents glycerol.

Figure 8 is a negative ion f.a.b.-mass spectrum of de-N-acetyl-GM₃ (D₁) in a triethanolamine matrix. Inset: molecular ion region of spectrum in a glycerol matrix.

Figure 9 is a negative ion f.a.b.-mass spectra of de-N-acetyl-lyso-GM₃ (D₂) in A: a triethanolamine matrix, B: a glycerol matrix.

Figure 10 is a negative ion f.a.b.-mass spectrum of lyso-GM₃ (D₃) in a triethanolamine matrix. Inset: molecular ion region of spectrum in a glycerol matrix.

For the purposes of this invention, the following terms have the following meanings:

Glycospingolipid -- The term "glycospingolipid" as used in this application indicates glycosphingolipids, which are the glycosides of N-acyl-sphingosine, the trivial name of which is ceramide. Sphingosines are a group of related long chain aliphatic 2-amino-1,3-di-hydroxy-(long chain bases), of which D-erythro-1,3-dihydroxy-2-amino-2,5-transoctadecene occurs most frequently.

Lyso form glyspingolipid -- Lyso-sphingolipids as the term is used in this application are sphingolipids in which N-fatty acyl groups linked to amino group of sphingosine are elimianted. Lyso-GM₃ is therefore sialyl-lactosylsphingosine or sialyl-Galβ1→4Glcβ1→sphingosine; lyso-GM₁ is Galβ1→3GalNAc-β1→4[sialyl-2→3]Galβ1→4Glcβ1→sphingosine, etc.

De-N-acylated glycospingolipid or lyso form glycospingolipid -- De-N-acylated glycosphingolipids or lyso glycospingolipids as used in this application are both de-N-fatty-acylated and the de-N-acetylated or de-N-glycolylated compounds. N-acetyl and N-glycolyl are originally linked at the sialic acid and N-acetyl linked at the hexosamine of the sugar chain.

Amino sugar-containing glycosphingolipid or lyso form glycospingolipid -- Amino sugar-containing glycosphingolipids and lyso glycospingolipids as used in this application are globo-series, lacto-series, and ganglio-series structures. Globo series contain N-acetyl-galactosamine at the terminus, ganglio series contain N-acetyl-galactosamine at the penultimate position, while lactoseries contain N-acetyl-glucosamine at the penultimate position. These are the simplest forms. Chains are further extended to create a large diversity of structures.

Ganglioside -- Glycospingolipids containing sialic acid (nonulosaminic acid or neuraminic acid containing an N-acetyl, N-glycolyl or O-acetyl group). The nomenclature used to designate ganglio-series ganglioside (e.g., GM₁, GM₂, GM₃, etc.) is according to Svennerholm, L., J. Neurochem., 10 (1963) 613-623.

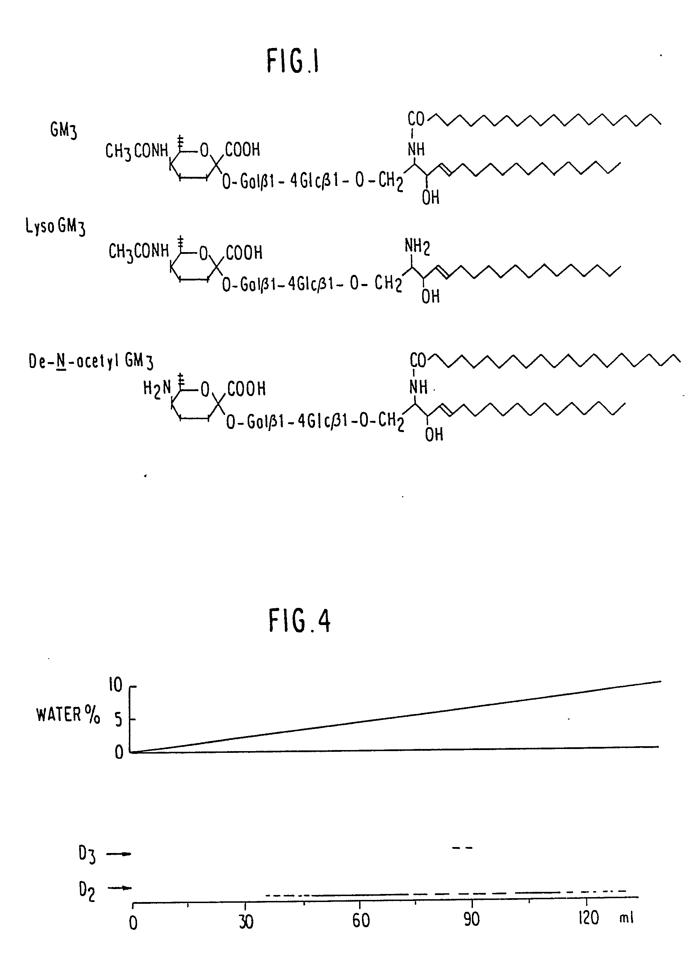

GM₃ -- A ganglioside having the structure shown in Fig. 1.

Lyso GM₃ -- A ganglioside having the structure shown in Figure 1.

De-N-Acetyl GM₃ -- A de-N-acetylated ganglioside having the structure shown in Fig. 1.

The novel methods of synthesizing the glycospingolipids according to the present invention will now be described in detail.

Isolation or preparation of glycospingolipid starting material

The synthesis of the de-N-acylated glycospingolipid and the acylated lyso form glycospingolipid according to the present invention can be accomplished by using the corresponding N-acylated glycospingolipid as the starting material.

General method for purification of N-acylated glycosphingolipids (in which the amino group of sphingosine is N-fatty acylated having C14-C24 aliphatic chains and the amino group of hexosamine in the sugar chain is N-acetylated and the amino group of neuraminic acid in the sugar chain is N-acetylated or N-glycolylated): The starting material as defined above, i.e., various types of gangliosides or amino sugar-containing glycosphingolipids, is prepared from natural sources after extraction with isopropanol-hexane-water, chloroform-methanol, or 90% ethanol, followed by various steps of purification, including Folch's partition, ion exchange chromatography in chloroform-methanol water, high pressure high performance liquid chromatography, etc., as described in Hakomori, S. (1983) In: Handbook of Lipid Research, Vol, 3, Sphingolipid Biochemistry (Kanfer, J.N., Hakomori, S., eds.), Plenum Publishing, New York, pp. 1-165. Glycosphingolipids can be synthesized by pure organic synthesis; however, the yield of such synthesis is extremely poor and involves a great many steps (see Hakomori, as above). Therefore, the starting material for preparation of the various derivatives preferably is not a synthetic compound.

When the desired modified glycospingolipid is a ganglioside, the synthesis of the de-N-acyl ganglioside and lyso form ganglioside can be accomplished by using the corresponding unmodified ganglioside containing N-acetyl and/or N-glycolyl neuraminic acid as the starting material.

The unmodified ganglioside containing N-acetyl and/or N-glycolyl neuraminic acid to be used as the precursor for the ganglioside containing de-N-acyl-sialic acid is obtained by the extraction from an appropriate source readily known to the skilled artisan (e.g. dog erythrocytes for GM3 and bovine brain for most other gangliosides) and purified by chromatography (R.K. Yu and R.W. Ledeen (1972) J. Lipid Res. 13, 680-686) on DEAE Sephadex followed by high-performance liquid chromatography on a column of latrobeads 6RS8010 using various gradient elution systems well known in the art (for example, see K. Watanabe and Y. Arao (1981) J. Lipid Res. 22, 1020-1024).

## Preparation of de-N-acyl glycospingolipid

The N-acyl sugar containing glycospingolipid prepared or isolated as described above is subjected to a critical step of hydrolysis such that the N-acyl group on the sugar moiety is preferentially hydrolyzed.

Specifically, the N-acyl group of the sugar moiety of the N-acyl sugar-containing glycospingolipid is preferentially hydrolyzed by treatment under mild alkaline conditions.

The mile alkaline conditions comprise using a base at a concentration of about 0.1M or below, and preferably the concentration of the base is about 0.1 M.

A suitable base that can be used is an alkali metal hydroxide and preferably the base is sodium hydroxide.

The reaction is conducted in a suitable solvent such as 90% 1-butanol at a temperature of about 80° C for about 4 hours.

The yield is generally greater than 70%.

After completeion of the reaction, the hydrolysate in the solvent is neutralized with acid (e.g., 12 M HCl) and taken to dryness. The residue is resuspended in water in any convenient volume and purified by an appropriate method such as by passing through a column of $C_{18}$ silica. The column is washed with water to eliminate salts and then the lipids, mostly glycospingolipids that have been de-N-acylated, are eluted with an appropriate solvent (e.g., methanol). The derivative can be purified by high-performance liquid chromatography.

## Preparation of lyso form glycospingolipid

In order to prepare the lyso form glycospingolipid, the N-acyl sugar containing glycospingolipid prepared or isolated as described above is deacylated and the fatty acid residue substituent on the amino group of the spingosine moiety removed to prepare an intermediate compound, specifically a de-N-acylated lyso glycospingolipid. One method by which this can be accomplished is by dissolving the glycolipid (about 6 μmol) in 1M KOH in aqueous 90% n-butanol and heating at about 117° C for about 2 hours.

Under these conditions, greater than 95% of the glycolipid is converted to the de-N-acyl lyso form (Taketomi, T. and Yamakawa, T., J. Biochem (Tokyo), 54 (1963) 444-451).

Next, the sugar moiety of the de-N-acyl lyso glycospingolipid is preferentially N-acylated by protecting the free amino group of the spingosine moiety.

Protection of the amino group of spingosine of de-N-acetyl lyso form glycospingolipids can be by PC-liposome or by any other type of phospholipid or hydrophobic matrix that interacts with the sphingosine moiety of the lyso form glycospingolipid. Dimyristoylor distearyl-PC, spingomyelin, diacetylphosphate, and any other synthetic compounds that offer hydrophobic interaction with sphingosine hydrocarbons of lyso form glycospingolipid analogs are satisfactory. This can be extended to solid-phase interaction of lyso form glycospingolipid analogs with a long chain alkylated silica gel matrix, which interacts with lyso form glycospingolipid analogs. In that case, the amino group of amino sugar or neuraminic acid can be N-acylated selectively. Lyso form glycospingolipid analogs, as mentioned above, include glycosylated sphingosine containing de-N-acetylated or de-N-glycolylated neuraminic acid or de-N-acetylated hexosamines (glucosamine or galactosamine).

Especially useful matrices are dp-PC liposomes.

In order to incorporate the de-N-acyl-lysospingoglycolipid into a matrix, the de-N-acyl-lyso-glyco spingolipid is mixed together with the phospholipid or hydrophobic substance in a molar ratio of about 1 to 10 in a suitable solvent and the mixture dried.

The de-N-acyl-lyso-glycosphingolipid/liposome complex is then resuspended by sonication, or other appropriate method, in a solution of DEC (dimethylaminopropylethylcarbodiimide) in water (about 20 mg DEC 1 ml $H_2O$). The suspension is cooled to about $4°C$ and the N-acylation reaction is initiated by adding an appropriate buffer (e.g., for N-acetylation, 0.1 M acetate buffer (about 0.2 ml, pH about 5.2/ml suspension)). The suspension is incubated for about 24 hours at about $4°C$ and the reaction stopped by the addition of ethanolamine (to about 20 μmol) followed by chloroform-methanol (2:1) (about 5 ml chloroform-methanol/1.2 ml suspension). Two phases are formed and the lower phase is washed with the same volume of chloroform-methanol-water (3:47:48 by vol). Two phases are again formed and the upper phases from each wash are combined, dried, resuspended in water, desalted (e.g., by passage through a $C_{18}$ silica column) and purified by high-performance liquid chromotography to give the lyso glycospingolipid in 70-80% yield.

## Preparation of labelled glycospingolipids

As mentioned above, the present invention also provides novel methods for producing labeled glycospingolipids.

## Preparation of labeled acylated forms of amino sugar-containing glycospingolipids labeled in the sugar moiety

According to the present invention a method is provided for producing an N-acylated glycospingolipid wherein the acyl group of the sugar moiety is labeled.

N-acylation reagents in general include N-acyl anhydride or N-acyl chloride or a mixture of protonated acyl group with carbodiimide catalysts. The acyl group can be labeled with [14C] and/or [3H]. The N-acylation reaction proceeds much more easily than an O-acylation reaction, and the yield is nearly 100%.

As one example, in order to prepare a radiolabeled N-acetylated glycospingolipid, an amino sugar-containing glycospingolipid which can be obtained, for example, by the method described above for preparing de-N-acylated glycolipids, can be N-acetylated by dissolving the amino sugar-containing glycolipid in an appropriate solvent containing DEC (dimethylaminopropylethylcarbodiimide)(2 mg/ml $H_2O$ for example), cooling to about $4°C$ and adding acetate buffer prepared from radiolabeled (e.g. [14C] or [3H]) acetic acid to a final concentration of about 0.02 M acetate. The mixture is incubated for about 24 hours at $4°C$ and the reaction stopped by addition of ethanolamine (to about 20 μmol). The radiolabeled compound is purified by high-performance liquid chromatography or other suitable method to give the radiolabeled glycolipid.

Alternatively, as another example, the de-N-acyl glycolipid can be N-acetylated with [14C] or [3H]-acetic anhydride as follows.

Dried samples of the de-N-acetyl glycolipid are dissolved in $NaHCO_3$ (about 0.5M) by stirring and heating at about $60°C$. [14C]-acetic anhydride in methanol is added in a volume to bring the $NaHCO_3$ concentration to about 0.1 M and the mixture is incubated for about 1 hour at about room temperature. After drying under a nitrogen stream, the reaction mixture is dissolved in distilled water and purified by an appropriate method such as by use of a $C_{18}$-silica gel column. Labeled glycolipid can be eluted with chloroform: methanol and analyzed by HPTLC through HPLC by latrobeads 6RS8010.

Preparation of labeled acylated glycospingolipids labeled in the fatty acid moiety

According to this method of the present invention, a fatty acylated glycospingolipid labeled in the fatty acid moiety can be prepared from an N-acylated lyso form glycospingolipid.

The N-acylated lyso form glycospingolipid can be prepared as described above.

The N-acylated lyso form glycospingolipid, after purification from the liposome or other matrix is dissolved in an appropriate solvent containing DEC and fatty acid is added which contains a labeled group. Alternatively an acyl chloride of fatty acids can be used as an acylating reagent of the amino group of spingosine. Suitable fatty acid residues for linking to the amino group of sphingosine include C14-C24 in normal composition. Mostly, C16-C18 for one group, and C20-C24 for another group. Further, depending on the requirement, an $\alpha$-hydroxylated fatty acid or any other substituted fatty acid, including radiolabeled, spin-labeled, or fluorescent-labeled group can be used. The skilled artisan can readily determine how to use these labeled fatty acids. The radiolabel can be, for example, $^{14}$C or $^{3}$H.

After incubating at an appropriate temperature for an appropriate amount of time, the thus formed fatty acid labeled glycospingolipid is separated from the reaction mixture and purified by high-performance liquid chromatography, or other suitable method to give the fatty acid labeled glycospingolipid.

An N-acetylated glycosphingolipid labeled in both the N-acetyl group of the sugar moiety and the fatty acid moiety can be produced according to the same methods except that the starting material is an N-acylated lyso form labeled in the N-acyl group on the sugar moiety produced according to the method described below.

Preparation of labeled N-acetylated lyso form glycospingolipids labeled in the N-acyl group on the sugar moiety

The method for preparing an N-acylated lyso form glycospingolipids labeled in the N-acyl group on the sugar moiety is analogous to the above-described method for preparing the lyso form glycospingolipids except that the N-acylation reaction is a reaction initiated by adding acetate buffer (about 0.1M) containing labeled (e.g. $^{14}$C or $^{3}$H) acetic acid or other suitable N-acylation reagents.

## EXAMPLES

The present invention will now be described by reference to specific examples which are not meant to be limiting.

Unless otherwise specified, all ratios, percents, etc. are by weight.

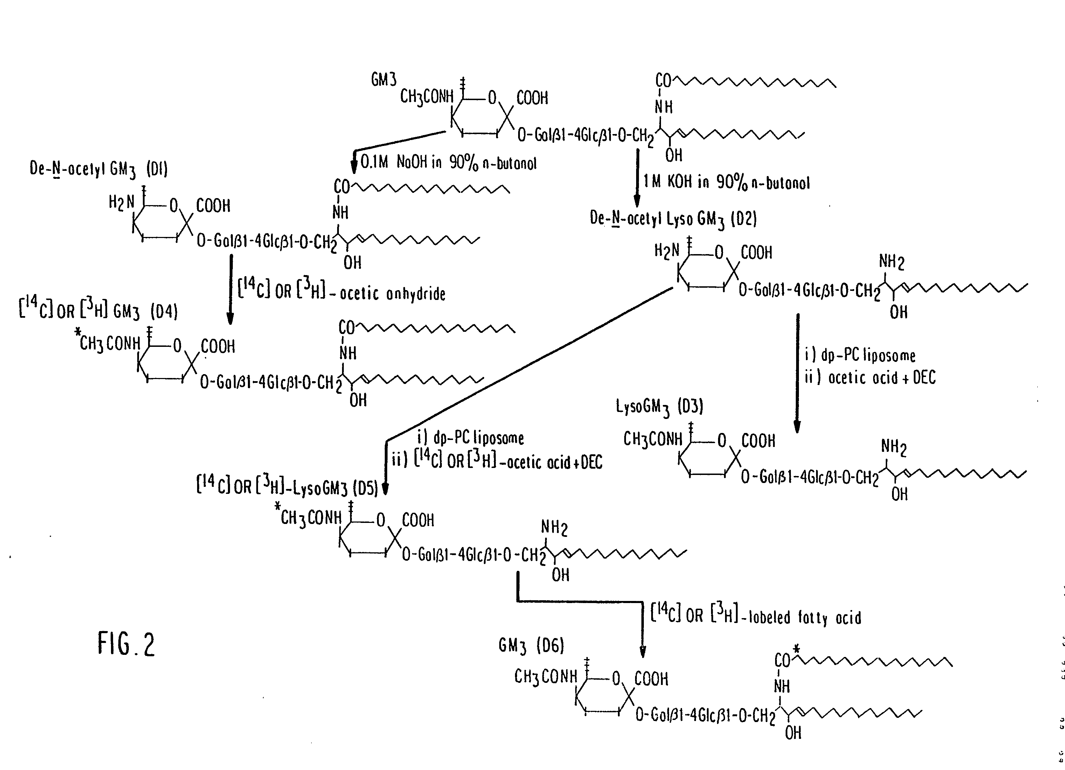

For clarity, reference can be made to Figure 2 which is a diagram of the reaction schemes for producing the glycolipids of the present invention.

## Example 1

### Synthesis of De-N-Acetyl Gm₃(D1)

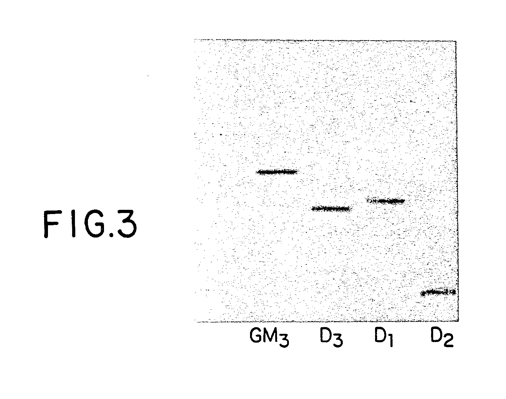

Isolation of GM₃ ganglioside -- GM₃ containing N-acetyl-neuraminic acid was extracted from dog erythrocytes and purified by chromatography (R.K. Yu and R.W. Ledeen, J. Lipid Res., 13 (1972) 680-686) on DEAE Sephadex followed by high-performance liquid chromatography on a column of latrobeads 6RS8010 in a 2-propanol-hexane-water system as previously described (K. Watanabe and Y. Arao J. Lipid Res. 22 (1981) 1020-1024). The behavoir on high-performance thin-layer chromatography is shown in Figure 3.

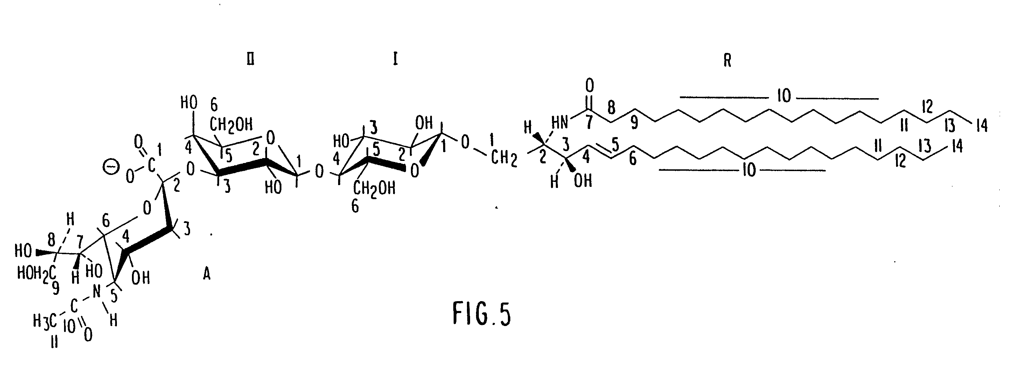

Preparation of de-N-acetyl-GM₃ -- On hydrolysis with 0.1 M NaOH in aqueous 90% 1-butanol at 80° for 4 hours, the N-acetyl group of GM₃ sialic acid was preferentially hydrolyzed to give de-N-acetyl-GM₃, which has a free amino group at the sialic acid moiety of GM₃ (See Figure 4), as the main product (≥70%). The hydrolysate in aqueous 1-butanol was neutralized with 12 M HC1, concentrated to dryness, and a solution of the residue in water (6 ml) was passed through a column of $C_{18}$ silica (Bond Elut, Analytichem International, Oxnard, CA). After washing with water to eliminate salts, the lipids were diluted with methanol. The derivatives were finally purified by high-performance liquid chromatography. The behavior on high-performance thin-layer chromatography is shown in Figure 3.

## Example 2

## Synthesis of N-[$^{14}$C]-Acetyl GM$_3$(D4)

De-N-acetyl-GM$_3$ was N-acetylated with [$^{14}$C]-acetic anhydride in methanol containing 0.1 M NaHCO$_3$ as follows.

Dried samples were dissolved in 50 $\mu$1 of 0.5 M NaHCO by stirring and heating at 60° C. Twenty-fife $\mu$Ci [$^{14}$C]-acetic anhydride (5.1 mCi/mmol) in 0.1 ml of methanol was added and the mixture was incubated for 1 hour at room temperature. After drying under a nitrogen stream, the reaction mixture was dissolved in distilled water and loaded on a $C_{18}$-silica gel column. Labeled glycolipid was eluted with chloroform:methanol (2:1, v/v) and analyzed by HPTLC (chloroform:methanol 0.02% CaC1$_2$-2H$_2$O, 60:40:9) through HPLC by latrobeads 6RS8010.

## Example 3

Synthesis of Lyso GM$_3$ (D3)

GM$_3$ containing N-acetyl-neuraminic acid was extracted from dog erythrocytes and purified by chromatography as described in Example 1 above.

Preparation of de-N-acetyl-lyso-GM$_3$ (D2) --De-N-acetyl-lyso-GM$_3$ is characterized by the absence of the N-acetyl group at the sialic acid moiety and the N-acyl group at the ceramide moiety of GM$_3$ (See Figure 2).

In order to prepare de-N-acetyl-lyso-GM$_3$ a solution of GM$_3$ (6 $\mu$mol) in 1 M KOH in aqueous 90% butanol (3 ml) was heated at 117° for 2 hours. Under these conditions, originally described by Taketomi and Yamakawa (T. Taketomi and T. Yamakawa (1963) J. Biochem. (Tokyo) 54, 444-451), more than 95% of the GM$_3$ was converted into de-N-acetyl-lyso-GM$_3$.

The behavior on high-performance thin-layer chromatography and in high-performance liquid chromatography is shown in Figures 3 and 4, respectively.

Synthesis of lyso-GM$_3$ (D$_3$) - The neuraminic acid residue of de-N-acetyl-lyso-GM$_3$ was preferentially N-acetylated by protecting the amino group of the sphingosine by inserting the de-N-acetyl-lyso-GM$_3$ in a liposome of dpPC (dipalmotoylphosphatidylcholine, obtained from Sigma Chemical Co.).

De-N-acetyl-GM$_3$ (1 $\mu$mol) was dried together with dpPC (10 $\mu$mol). A solution of 20 mg of DEC (aminopropylethylcarbodiimide, obtained from Aldrich Chemical Co.) in water (1 ml) was added, and the lipids were resuspended by sonication with the needle probe of a sonicator (Braun-Sonic 1510) using 30 watts of power for about 5 minutes. The suspension was cooled to 4° and the N-acetylation reaction was initiated by adding 0.1 M acetate buffer (0.2 ml, pH 5.2). After incubation for 24 hours at 4°, the reaction was stopped by the addition of ethanolamine (20 $\mu$mol) followed by chloroform-methanol (5 ml. 2:1). The lower phase was washed with the same volume of chloroform-methanol-water (3:47:48 v:v:v), and the combined upper phases were dried, resuspended in water, desalted by passage through a $C_{18}$ silica column and purified by HPLC to give lyso-GM$_3$ (70-80%). The behavior on high-performance thin layer chromatography and in high-performance liquid chromatography is shown in Figures 3 and 4, respectively.

## Example 4

## Characterization of GM$_3$ Derivatives D1, D2 and D3

The derivatives were characterized by NMR spectroscopy and negative ion fast atom bombardment (f.a.b.) spectrometry. Solutions of the compound (400 μg) in Me$_2$SO-d$_6$-D$_2$O (98:2, 0.3 ml) were stored for 5 minutes to allow deuterium exchange of hydroxy and amino protons. Each solution was then lyophilized and a solution of the residue Me$_2$SO-d$_6$-D$_2$O (98:2, 0.5 ml) was used immediately for NMR spectroscopy. $^1$H-NMR spectra were obtained at 35° with a Bruker WM-500 spectrometer equipped with an Aspect 2000 computer using quadrature detection, a spectral width of 5000 Hz over 16K data points, and a relaxation delay of 2 s. The number of transients collected varied from 200 to 500. Chemical shifts are referenced to the terminal methyl resonance(s), the shift of the resonance of which was assumed to be 0.85 p.p.m.

The structure and proton labeling scheme for GM$_3$ de-N-acetyl-GM$_3$ (D$_1$), de-N-acetyl-lyso-GM$_3$ (D$_2$) and lyso-GM$_3$ (D$_3$) are shown in Figure 5.

Negative ion f.a.b.-mass spectrometry was performed using a JEOL HX-110 mass spectrometer/DA-5000 data system. Solutions of samples in methanol were transferred to a triethanolamine or glycerol matrix on the f.a.b. target and bombarded with a xenon beam. The acceleration voltage was 10 kV and the resolution was 3000. Data were acquired in the accumulation mode from 100-1500 a.m.u. (atomic mass units) with a scan slope of 1 min/decade. Each spectrum represents the accumulation or three scans. Sodium iodide in glyceral was used as the mass calibration standard.

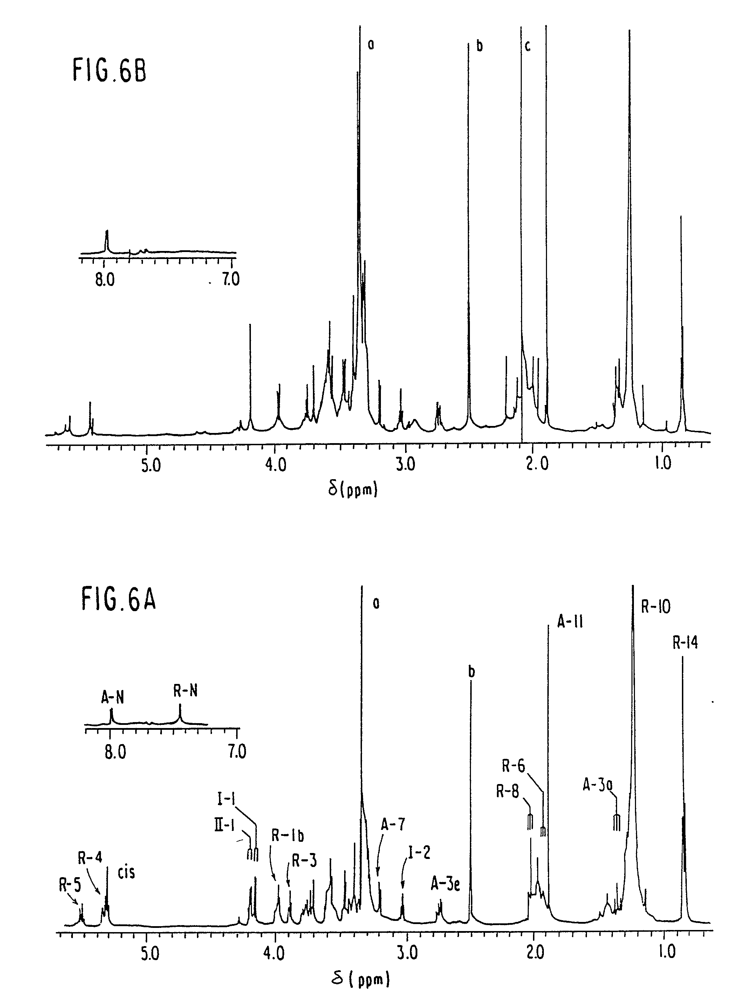

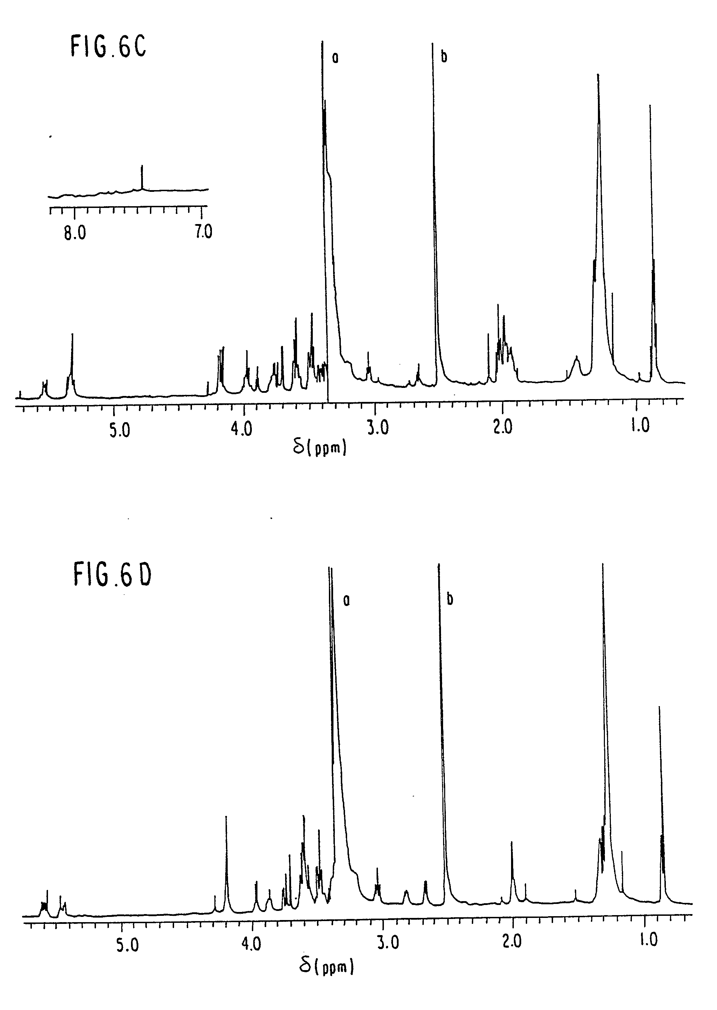

Figures 6A, 6B, 6C and 6D show the $^1$H-NMR spectra of GM$_3$, D$_3$, D$_1$ and D$_2$, respectively, along with selected resonance assignments.

The spectrum of GM$_3$ (Figure 6A) is identical to that previously reported (T.A.W. Koerner Jr., et al, Biochemistry, 22 (1983) 2676-2690) and the assignments follow those of these authors, except for the of the amide protons, which were not assigned (T.A.W. Koerner Jr., et al, Biochemistry, 22 (1983) 2676-2690). The amide assignments were made by comparison with the spectra of de-N-acetyl-Gm$_3$ (D$_1$) and lyso-GM$_3$ -(D$_3$), and agree with the assignments for GM$_3$ in Me$_2$SO-d$_6$ (deuterated dimethyl sulfoxide) at 35° (S. Gasa et al J. Lipid Res., 24 (1983) 174-182).

Of special note for the identification of GM$_3$ derivatives are the resonances for the cis olefinic protons of the unsaturated fatty acid (δ 5.3), the anomeric protons of glucose (I-1, δ 4.17) and galactose (II-1, δ 4.19), NAc of the sialic acid (A-11, δ 1.88), and the amide protons R-N and A-N (δ 7.45 and 7.98, respectively).

Comparison of the spectra of lyso-GM$_3$ (D$_3$) Figure 6B) and GM$_3$ shows that the former lacks the resonances (δ 5.3) due to the cis olefinic protons of the unsaturated fatty acid and the amide proton (δ 7.45). Loss of the fatty acid from sphingosine was verified by integration of the methylene (R-10) and terminal methyl (R-14) resonances (δ 1.25 and 0.85, respectively). Another notable difference between the spectra of GM$_3$ and lyso-GM$_3$ is the down-field shift (0.032 p.p.m.) for the resonance of the glucose anomeric proton (I-1) in the spectrum of lyso-GM$_3$. This significant substituent effect is most likely caused by loss of the anisotropic shielding of the fatty acid carbonyl. The change in the chemical shift of the resonance of the glucose anomeric protein upon loss of the fatty acid implies a relatively close approach of the fatty acid carbonyl to the glucose anomeric proton in GM$_3$, and may have implications for the secondary structure of GM$_3$. Other differences between the spectra of Figures 6A and 6B are evident and are compatible with the proposed structure of lyso-GM$_3$; the sphingosine olefinic proton resonances R-4 and R-5 (δ 5.3 and 5.5, respectively) are shifted down-field in Figure 6B compared to their positions in Figure 6A. In addition, the multiplicities and chemical shifts of the resonances of the sphingosine protons R-1b and R-3 (δ 3.96 and 3.88, respectively) change upon loss of the fatty acid bound to sphingosine.

Figure 6C shows the spectrum of de-N-acetyl-GM$_3$ (D$_1$) which differs from that of GM$_3$ by the absence of the resonances for the sialic acid acetamido methyl group (A-11, δ 1.89) and the sialic acid amide proton (A-N, δ 7.98). In addition, the resonance for sialic acid H-7, which appears at δ 3.1 in the spectrum of GM$_3$, moved down-field in the spectrum of de-N-acetyl-GM$_3$, whereas that of H-3e moved up-field (-0.1 p.p.m.). These shifts may be explained by loss of the anisotropic effect of the carbonyl of the acetamido group formerly bound to neuraminic acid.

The spectrum of de-N-acetyl-lyso-GM$_3$ (D$_2$) appears in Figure 6D. The spectrum of de-N-acety-lyso-GM$_3$ contains no resonance for NAc.

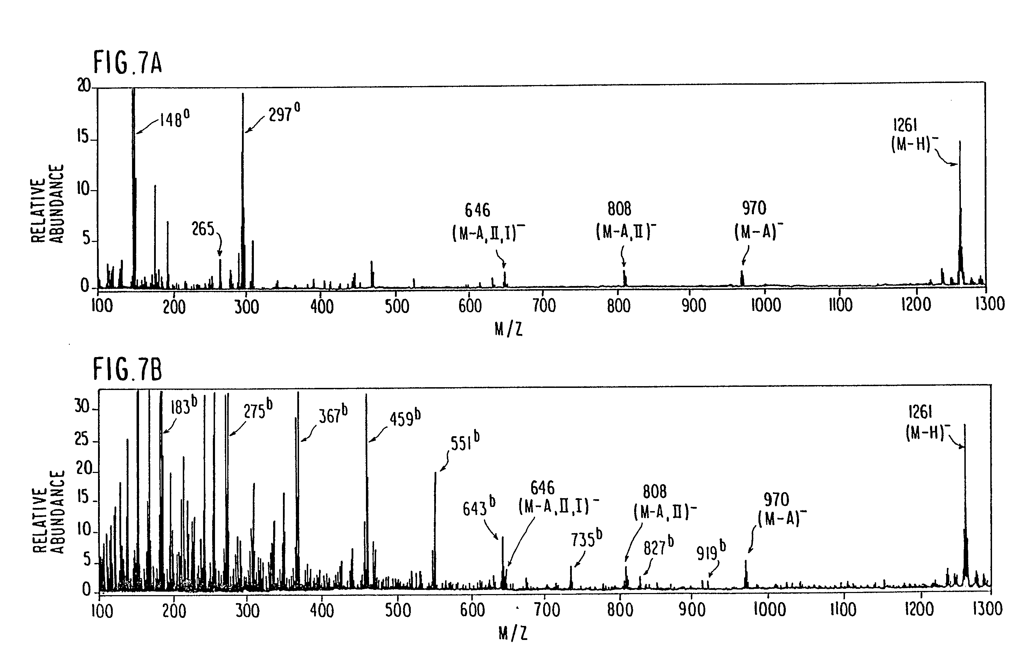

The major fragments observed in the mass spectra of GM$_3$ and D$_1$-D$_3$ obtained in both triethanolamine and glycerol matrices are summarized in Table I and the spectra are shown in Figures 7-10, respectively.

**TABLE I**

**Mass (m/z) of relevant fragments produced by negative ion f.a.b.-mass spectrometry of $GM_3$ and synthetic derivatives**

$$\begin{array}{ccc}
\overbrace{\phantom{M-A}}^{M-A} & \overbrace{\phantom{M-A,II}}^{M-A,II} & \overbrace{\phantom{M-A,II,I}}^{M-A,II,I}
\end{array}$$

Neu—O—Gal—O—Glc—O—$CH_2CHCHOHCH=CH(CH_2)_{12}CH_3$

with NH—$R_1$ on Gal and NH—$R_2$ on the ceramide

    A     II     I

| | $R_1$ | $R_2$ | M-H | M-A | M-A,II | M-A,II,I |
|---|---|---|---|---|---|---|
| $GM_3$ | $COCH_3$ | $COC_{23}H_{45}$ | 1261 | 970 | 808 | 646 |
| $D_1$ | H | $COC_{23}H_{45}$ | 1219 | 970 | 808 | 646 |
| $D_2$ | H | H | 871 | 622 | 460 | 298[a] |
| $D_3$ | $COCH_3$ | H | 913 | 622 | 460 | 298[a] |

[a] Obscured by the triethanolamine matrix cluster ion at m/z 297.

The negative ion f.a.b.-mass spectrum of $GM_3$ in a triethanolamine matrix (Figure 7A) was dominated by the pseudo-molecular ions, some fragmention at occurring through loss of sugar residues from the non-reducing end, with charge retention on the ceramide-containing portion of the molecule. The presence in canine erythrocyte $GM_3$ of ceramide composed almost exclusively of d18:1 sphinogosine and 24:1 (+24:0) fatty acid, was indicated by the abundant ion at m/z 1261 (1263). The fragment expected from d18:1 sphingosine, at m/z 237, was not observed in the spectrum.

In the spectrum obtained using a glycerol matrix (Figure 7B), the same ions were observed (m/z 1261, 970, 808, 646), along with a number of glycerol cluster ions (nG-1)⁻. The spectra of the mono-de-N-acyl compounds lyso-$GM_3$ ($D_3$) and de-N-acetyl-$GM_3$ ($D_1$) in a triethanolamine matrix were characterized by the unexpected presence, in addition to the abundant pseudomolecular (M-H)⁻ ions, of ions 26 and 42 a.m.u. higher in mass. Since the (M-H+42)⁻ ions coincide with the mass of (M-H)⁻ expected for other synthetic derivatives which are N-acetylated, this finding was first interpreted as an indication of mutual contamination. However, the preparation and purification schemes seemed to exclude this possibility and, in addition, the ¹H-NMR spectra did not corroborate the presence of mixtures. Moreover, the production of (M-H+26)⁻ ions could not be explained simply as due to impurities, nor could the similarities in the relative abundances of these molecular weight region clusters. Finally, the occurrence of a second set of higher mass ions [(M-H+42+16)⁻ and (M-H+42+42)⁻] in the spectrum of de-N-acetyl-lyso $GM_3$ ($D_2$) (discussed further below), which is deacylated at both nitrogens, seemed to indicate that the presence of these ions is related to the number of free amino functions in the molecule, and that they are the result of some reaction taking place during the bombardment process in a triethanolamine matrix. In order to assess this possibility spectra were aquired in another matrix, glycerol. The results appear to confirm this hypothesis, as well as the identity and purity of the synthetic ganglioside derivatives.

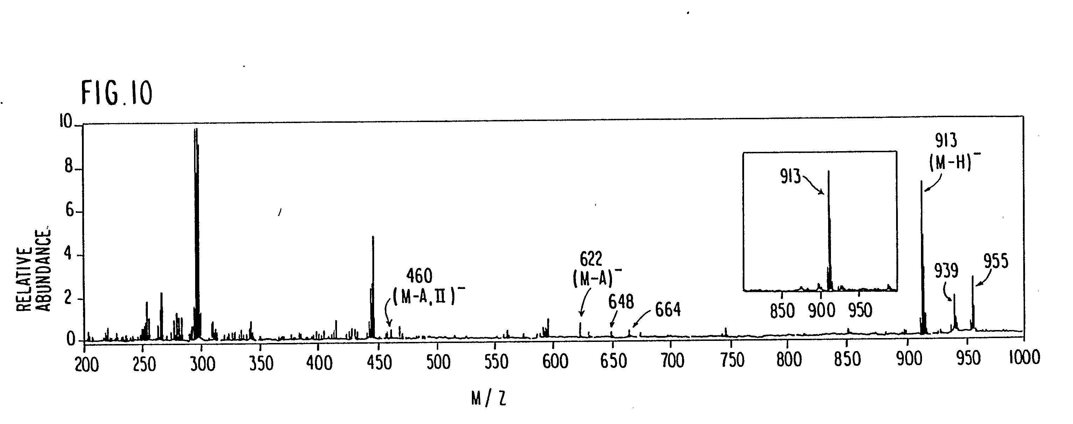

Thus, whereas the spectrum of lyso-$GM_3$ ($D_3$) in a triethanolamine matrix (Figure 10) contained abundant ions at m/z 913, these extra ions were absent in the spectrum obtained in a glycerol matrix (figure 10, inset). The spectrum of lyso-GM$_3$ in a triethanolamine matrix also contained ions corresponding to loss of NeuAc (m/z 622) and of NeuAc•Hex (m/z 460). The ion produced by loss of NeuAc and both hexoses, corresponding to (sphingosine-H)$^-$ and expected at m/z 298, was obscured by the large matrix cluster ion at m/z 297. Inspection of the spectrum of lyso-GM$_3$ in a triethanolamine matrix revealed ions at m/z 648 and 664, corresponding to the loss of NeuAc from the extra ions at m/z 939 and 955, respectively. This result might be expected from a chemical reaction involving a free amino group on sphingosine. The f.a.b.-mass spectrum for a glycerol matrix showed little useful fragmentation.

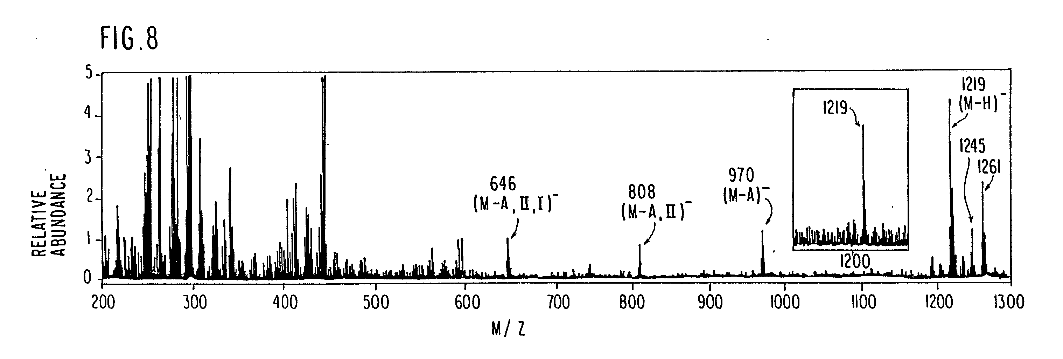

The de-N-acetylation of GM$_3$ NeuAc to produce de-N-acetyl-GM$_3$ (D$_1$) should reduce the mass of the pseudomolecular ions by 42 a.m.u. In the mass spectrum of de-N-acetyl-GM$_3$ in a triethanolamine matrix (Figure 8), the expected predominant pseudomolecular ion was found at m/z 1219 (1221) for 24:1 (+24:0) fatty acid containing ceramides, accompanied, as for lyso-GM$_3$, by associated (M-H+26)$^-$ and (M-H+42)$^-$ ions at m/z 1245 (1247) and 1261 (1263). As with lyso-GM$_3$, the mass spectrum of de-N-acetyl-GM$_3$ in a glycerol matrix (Figure 8, inset) exhibited only the pseudomolecular ions (m/z 1219, 1221), again with little useful fragmentation. The abundant fragment ions seen with the triethanolamine matrix (m/z 970, 972, 808, 810, 646, 648) were unaccompanied by additional higher mass clusters, since the terminal neuraminic acid possessing the free amino function was eliminated in the production of these fragments. The fact that the difference between (M-H)$^-$ and (M-A)$^-$ is 249 a.m.u., instead of 291 a.m.u. expected for loss of NeuAc, confined the missing 24 a.m.u. to that residue, confirming its de-N-acetylation.

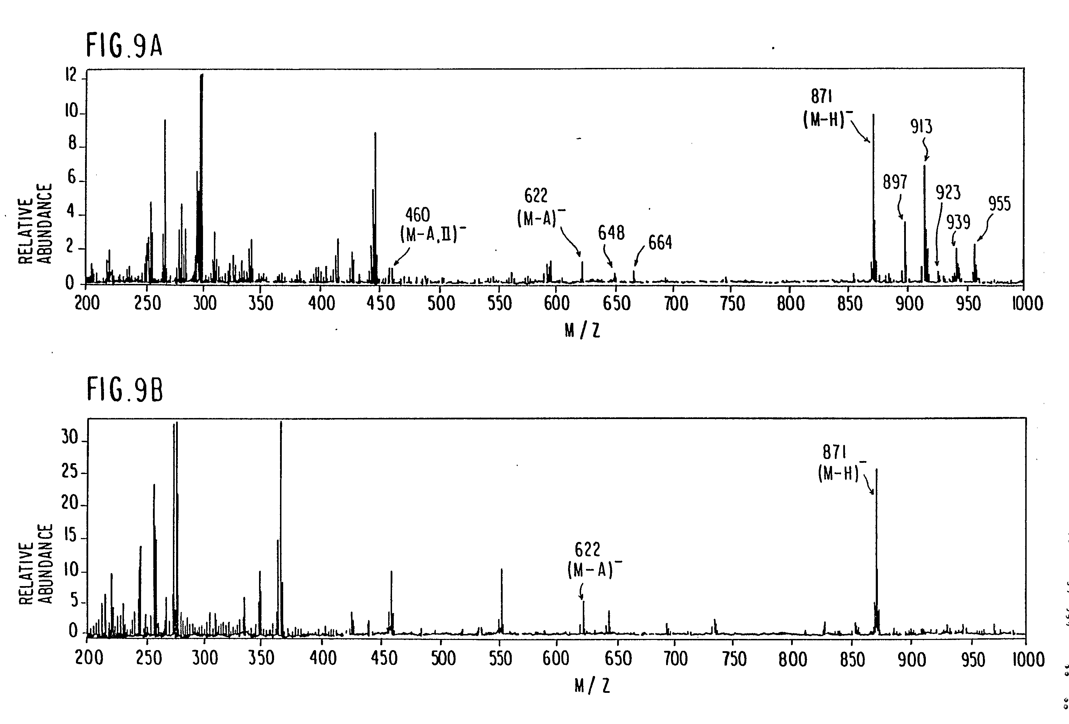

In the spectrum of the di-de-N-acylated ganglioside, i.e., de-N-acetyl-lyso-GM$_3$ (D$_2$) taken in a triethanolamine matrix (Figure 9A), the expected pseudomolecular ion at m/z 871 was accompanied by a series of abundant ions representing (M-H+26)$^-$, (M-H+42)$'$, (M-H+42+26)$^-$, and (M-H+42+42)$^-$. A less abundant ion, representing (M-H+26+26)$^-$, could also be detected. With a glycerol matrix, only the pseudomolecular ion could be seen (Figure 9B). The ion representing elimination of NeuNH$_2$ was present in the spectra for both the triethanolamine and glycerol matrices at m/z 622, and in former only was this accompanied by the higher mass ions m/z 648 and 664, as found for lyso-GM$_3$. The fragment representing loss of NeuNH$_2$•Hex (m/z 460) was detectable for the triethanolamine matrix, but its presence in the glycerol matrix was obscured by a matrix cluster ion at m/z 459.

While the invention has been described in detail and with reference to specific embodiments thereof, it will be apparent to one skilled in the art that various changes and modifications can be made therein without departing from the spirit and scope thereof.

## Claims

1. A method for preparing de-N-acylated forms of an N-acyl sugar-containing glycospingolipid comprising hydrolyzing the glycospingolipids under mild alkaline conditions such that the N-acyl group of the sugar moiety is preferentially hydrolyzed.

2. The method of Claim 1, wherein the N-acyl group of the sugar is an N-acetyl group or N-glycolyl group.

3. The method of Claim 1, wherein the glycospingolipid is a ganglioside.

4. The method of Claim 3, wherein the N-acyl group is an N-acetyl group or N-glycolyl group of sialic acid.

5. The method of Claim 4, wherein the ganglioside is GM$_3$.

6. The method of Claim 1, wherein the mild alkaline conditions comprise using a base at a concentration of about 0.1 M or below.

7. The method of Claim 6, wherein the base is an alkali metal hydroxide.

8. The method of Claim 7, wherein the base is NaOH.

9. The method of Claim 8, wherein the NaOH is present in a concentration of about 0.1 M.

10. A method for preparing labeled N-acylated forms of amino sugar-containing glycospingolipids comprising N-acylating the amino group on the sugar moiety with a labeled N-acylation reagent.

11. The method of Claim 10, wherein the amino sugar-containing glycospingolipid is obtained by hydrolyzing a N-acyl sugar-containing glycospingolipid under mild alkaline conditions such that the N-acyl group of the sugar moiety is preferentially hydrolyzed.

12. The method of Claim 10, wherein the labeled N-acylation reagent is an N-acetylation reagent.

13. The method of Claim 12 wherein the N-acetylation reagent is [$^{14}$C]- or [$^3$H]-acetic acid or its anhydride.

14. The method of Claim 10, wherein the amino sugar-containing glycospingolipid is a de-N-acetyl-ganglioside.

15. The method of Claim 14, wherein the de-N-acetyl-ganglioside is GM₃.

16. A method for preparing N-acylated forms of an amino sugar-containing or neuraminic acid-containing lyso form glycospingolipid comprising protecting the amino group of the spingosine by incorporating the glycospingolipid into a phospholipid or hydrophobic matrix and then conducting selective N-acylation on the amino group of the sugar moiety.

17. The method of Claim 16, wherein the amino sugar-containing lyso form glycospingolipid is produced by hydrolyzing an N-acyl amino sugar-containing or sialic acid-containing glycospingolipid wherein the amino group of the spingosine moiety is substituted with a fatty acid under alkaline conditions such that the N-acyl group of the amino sugar moiety and the substituted amino group of spingosine moiety are hydrolyzed.

18. The method of Claim 16, wherein the matrix is a liposome.

19. The method of Claim 18 wherein the liposome is a dipalmitoylphosphatidylcholine liposome.

20. The method of Claim 16 wherein the acylation is acetylation.

21. The method of Claim 16, wherein the amino sugar-containing lyso form glycospingolipid is de-N-acetyl lyso GM₃.

22. A method of preparing labeled N-acylated forms of an amino-sugar containing lyso form glycospingolipid comprising protecting the amino group of the spingosine by incorporating the glycospingolipid into a phospholipid or hydrophobic matrix and then conducting selective N-acylation on the amino group of the sugar moiety with a labeled N-acylation reagent.

23. The method of Claim 22, wherein the amino sugar-containing lyso form glycospingolipid is produced by hydrolyzing an N-acyl sugar-containing glycospingolipid wherein the amino group of the spingosine moiety is substituted with a fatty acid under alkaline conditions such as that the N-acyl group of the sugar moiety and the substituted amino group of the spingosine moiety are hydrolyzed.

24. The method of Claim 22, wherein the matrix is a liposome.

25. The method of Claim 24, wherein the liposome is a dipalmitoylphosphatidylcholine liposome.

26. The method of Claim 22, wherein the radiolabeled N-acylation reagent is an N-acetylation reagent.

27. The method of CLaim 26, wherein the N-acetylation reagent is [¹⁴C]-or [³H]-acetic acid.

28. The method of Claim 22, wherein the amino sugar-containing lyso form glycospingolipid is a de-N-acetyl lyso ganglioside.

29. The method of Claim 28, wherein the de-N-acetyl lyso ganglioside is a de-N-acetyl lyso GM₃.

30. A method for preparing labeled glycospingolipids from an acylated sugar-containing lyso form glycospingolipid comprising acylating the amino group on the spingosine moiety with a fatty acid containing labeled groups.

31. The method of Claim 30, wherein the acylated sugar-containing lyso form glycospingolipid is produced by protecting the amino group of the spingosine by an incorporating amino sugar-containing lyso form glycospingolipid into a phospholipid or hydrophobic matrix, conducting selective N-acylation on the amino group of the sugar moiety, and then isolating the lyso form glycolipid from the matrix.

32. The method of Claim 30, wherein the radiolabeled acylating reagent is an acetylating reagent.

33. The method of Claim 32, wherein the acetylating reagent is [¹⁴C]- or [³H]- acetic acid or its anhydride.

34. The method of Claim 30, wherein the acylated sugar-containing lyso form glycospingolipid is a lyso ganglisoide.

35. The method of Claim 34, wherein the lyso ganglioside is lyso GM₃.

36. The method of Claim 30, wherein the fatty acid containing an end group derived from a radiolabeled acylating reagent is one member selected from the group consisting of an $\alpha$-hydroxylated $C_{14}$-$C_{24}$ fatty acid, $C_{16}$-$C_{18}$ fatty acid and $C_{20}$-$C_{24}$ fatty acid.

# FIG.1

GM3

Lyso GM3

De-N-acetyl GM3

# FIG.4

FIG. 2

EP 0 332 298 A2

FIG.3

GM₃    D₃    D₁    D₂

FIG.5

EP 0 332 298 A2

FIG.6B

FIG.6A

FIG.6C

FIG.6D

FIG.7A

FIG.7B

EP 0 332 298 A2

FIG.8

EP 0 332 298 A2

RELATIVE ABUNDANCE

646
(M-A,II,I)⁻

808
(M-A,II)⁻

970
(M-A)⁻

1219
(M-H)⁻

1245

1261

1219

1200

M/Z

FIG.9A

FIG.9B

EP 0 332 298 A2

FIG.10

EP 0 332 298 A2